# EUROPEAN PATENT APPLICATION

(11) **EP 1 891 982 A2**
(43) Date of publication of application: **27.02.2008**
(21) Application number: 07016363.9
(22) Date of filing: 21.08.2007
(51) Int. Cl.: A61L 9/14, F24F 3/16

(54) **Air filtering apparatus having foreign material removing mechanism**

(30) Priority: 22.08.2006 JP 2006224951; 28.08.2006 JP 2006229989
(71) Applicant: Sanyo Electric Co., Ltd., Osaka 570-8677 (JP)
(72) Inventor: Fukushima, Toshio, Ota-shi, Gunma 373-0038 (JP); Arakawa, Toru, Tatebayashi-shi, Gunma 374-0066 (JP); Kobayashi, Hiroyuki, Ora-gun, Gunma 373-0533 (JP); Usui, Hiroaki, Ora-gun, Gunma 370-0523 (JP); Uchida, Yoichi, Ashikaga-shi, Tochigi 326-0006 (JP); Ogura, Nobhiro, Kiryu-shi, Gunma 376-0034 (JP); Kurokawa, Keiko, Ora-gun, Gunma 370-0517 (JP); Suzuki, Daisuke, Ota-shi, Gunma 373-0817 (JP); Kuribara, Hiroyuki, Ota-shi, Gunma 373-0817 (JP); Nakata, Yuji, Ora-gun, Gunma 370-0536 (JP); Yamamoto, Tetsuya, Ota-shi, Gunma 373-0806 (JP)
(74) Representative: Glawe, Delfs, Moll

(57) **Abstract**

An air filtering apparatus (100) for filtering air by using electrolytic including a housing (10) having an air suction port (11) and air blow-out port (12), an air blower (30) forming an air flowing passage extending from the air suction port (11) to the air blow-out port (12) in the housing, an air filtering unit (40) that is disposed on the air flowing passage and brings air introduced into the housing (10) into contact with electrolytic water to filter the air and a foreign material removing mechanism (20, 121) that is disposed on the air flowing passage and removes foreign materials contained in the air.

## Description

### BACKGROUND OF THE INVENTION

### 1. Field of the Invention

The present invention relates to an air filtering apparatus that can remove (inactivate, sterilize or the like) microorganisms such as bacteria, virus, fungus, etc. (hereinafter referred to as "virus, etc.") floating in the air.

### 2. Description of the Related Art

For the purpose of removing virus, etc. floating in the air, there is generally proposed an air filtering apparatus in which tap water or the like is electrolyzed to generate electrolytic water containing active oxygen species such as hypochlorous acid or the like, the thus generated electrolytic water is supplied to a humidifying element (filter, gas-liquid contact member) formed of non-woven cloth or the like, and air is supplied to the humidifying element and brought into contact with the electrolytic water to inactivate virus, etc. contained in the air, thereby filtering the air (for example, JP-A-2002-181358).

In addition to extremely small particulate materials such as microorganisms floating in the air, indoor air contains particulate materials having larger particle sizes than the microorganisms floating in the air, for examples, pollens such as cedar pollen, etc., house dusts such as lint, etc., sand, dirt, etc. (hereinafter referred to as "pollen etc."), and also gaseous materials such as odor components, etc. The particulate materials of pollen, etc. are generally larger in particle size (for example, 10µm or more) and more liable to be precipitated as compared with the microorganisms such as virus, etc. floating in the air. However, these materials are blown up by air stream and they may invade into human bodies through breadth or the like. The pollen, etc. induce symptoms such as allergic symptoms or the like, and thus it is preferable that these relatively large particulate materials are removed (filtered) from indoor air.

Furthermore, the conventional air filtering apparatus is equipped with an air blow-out port from which air passed through the humidifying element (gas-liquid contact member) is blown out to the outside of the apparatus. This air blow-out port is opened at all times, and thus there is concern that foreign materials such as pollen, etc. invade from the outside through the air blow-out port into the housing when the air blowing operation is stopped. In addition to the above concern on the invasion of foreign materials, the inside of the apparatus may be stained with the use of the air filtering apparatus, so that maintenance such as cleaning of the inside of the housing must be periodically conducted and thus the maintenance cost is increased.

### SUMMARY OF THE INVENTION

An object of the present invention is to provide an air filtering apparatus that can prevent contamination of foreign materials into an air flowing passage to reduce the frequency of maintenance.

In order to attain the above obj ect, according to the present invention, an air filtering apparatus (100) for filtering air by using electrolytic water comprises:
a housing (10) having an air suction port (11) and air blow-out port (12);
an air blower (30) forming an air flowing passage extending from the air suction port (11) to the air blow-out port (12) in the housing;
an air filtering unit (40) that is disposed on the air flowing passage and brings air introduced into the housing (10) into contact with electrolytic water to filter the air; and
a foreign material removing mechanism (20, 121) that is disposed on the air flowing passage and removes foreign materials contained in the air.

According to the above air filtering apparatus, the air flowing passage is formed by the air blower so as to extend from the air suction port to the air blow-out port. The air filtering unit is disposed on the air flowing passage, and the air and the electrolytic water which are introduced into the housing are brought into contact with each other on a gas-liquid contact face, thereby filtering the air.

The foreign material removing mechanism is disposed on the air flowing passage, and removes foreignmaterials contained in air introduced through the air flowing passage into the housing. Therefore, for example, by disposing the foreign material removing mechanism at the upstream side of the air filtering unit with respect to the air flowing direction, the foreign materials contained in the air flowing to the air filtering unit can be removed, so that the air can be efficiently filtered in the air filtering unit and the frequency of maintenance of the air filtering unit can be reduced. Furthermore, if the foreign material removing mechanism is disposed in the neighborhood of the air blow-out port on the air flowing passage, foreign materials can be prevented from invading through the air blow-out port into the housing, and thus adhesion of stains to the inside of the apparatus can be prevented, so that the frequency of the maintenance can be reduced.

Furthermore, in the above air filtering apparatus, the foreign material removing mechanism (20) is equipped with a pre-filter (2) for collecting materials each of which is contained in air introduced into the housing and has a predetermined particle size or more, and the air filtering unit (40) has a gas-liquid contact member (41) that is disposed at the downstream side of the pre-filter (20) on the air flowing passage and has a gas-liquid contact face, the air from which the materials each having the predetermined particle size or more are removed being brought into contact with the electrolytic water to thereby filter the air.

According to the above air filtering apparatus, the air blower forms the air flowing passage extending from the air suction port to the air blow-out port in the housing. The pre-filter is disposed at the upstream side of the air flowing passage with respect to the air flowing direction, and the materials whose particle sizes are equal to a predetermined value or more are collected by the pre-filter. Furthermore, the air filtering unit is disposed at the downstream side of the pre-filter with respect to the air flowing direction, and the air that is passed through the pre-filter to remove the materials of the predetermined particle size or more from the air and thus contains materials which have particle sizes less than the predetermined particle size and are hardly precipitated is brought into contact with the electrolytic water containing the active oxygen species.

Accordingly, with respect to particulate materials floating in the air which have relatively large particle sizes (particle diameters) and thus are liable to be precipitated, for example, house dust such as lint, etc., pollens such as cedar pollen, etc., dust of sand, soil, etc., large fungus of spore, etc., these materials can be collected by the pre-filter. Furthermore, with respect to particulate materials floating in the air (floating fine particles) which are small in particle size and thus hardly precipitated, for example, so-called indoor suspended particles of dust which floats indoor for a longtime, floating microorganisms such as virus, etc. floating in the air while adhering to indoor suspended particles of dust, gaseous materials such as odor components, etc., fractured pollens floating in the air, etc., they can be efficiently removed (inactivated, sterilized, etc.) from the air by using electrolytic water containing active oxygen species, thereby filtering the air.

Furthermore, with respect to the pre-filter, floating particulate materials of a predetermined particle size or more (hereinafter referred to as"large-size particulate materials") out of all the floating particulate materials are collected by the filter. Therefore, air can be efficiently sucked from the air suction port and then blown out from the air blow-out port by the air blower with little pressure loss. Furthermore, the large-size floating particulate materials are removed from the air by the pre-filter. Therefore, as compared with a case where all the floating particulate materials of all the particle sizes are removed from the air by the air filtering unit, the consumption of the electrolytic water can be reduced, and the maintenance frequency of the air filtering unit can be reduced.

In the above air filtering apparatus, the predetermined particle size is preferably equal to about 10µm. That is, the particle passing size (diameter) of the pre-filter is preferably set to about 10µm or less.

In the above air filtering apparatus, it is preferable that the housing is designed as an on-floor mount type, the air suction port is formed at the lower portion of the housing and the air blow-out port is formed at the upper portion of the housing.

In the above air filtering apparatus, it is preferable that the pre-filter (20) is disposed inside the air suction port (11).

In the above air filtering apparatus, it is preferable that the pre-filter comprises a water-washable filter so that collected foreign materials are removed.

It is preferable that the above air filtering apparatus further comprises an electrolytic water supply unit (50) for supplying electrolytic water to the gas-liquid contact face of the gas-liquid contact member (41.

In the above air filtering apparatus, it is preferable that the electrolytic water supply unit (50) is equipped with an electrolytic bath having at least a pair of electrodes (53a, 53b) for electrolyzing water or water containing predetermined ion species introduced into the electrolytic bath to generate electrolytic water containing active oxygen species.

In the above air filtering apparatus, the active oxygen species preferably contains at least one material selected from the group consisting of hypochlorous acid, hypoiodous acid, ozone and hydrogen peroxide.

In the above air filtering apparatus, it is preferable that the foreign material removing mechanism (20) has a pre-filter through which easily-precipitable materials out of all materials contained in air introduced into the housing are collected and hardly-precipitable materials out of all the materials are passed; and the air filtering unit (40) has a gas-liquid contact member (41) that is disposed at the downstream side of the pre-filter(20) on the air flowing passage, has a gas-liquid contact face and brings air passed through the pre-filter into contact with the electrolytic water on the gas-liquid contact face, thereby removing the hardly-precipitable materials from the air

According to the air filtering apparatus of the present invention, it is equipped with the housing having the air suction port and the air blow-out port, the air blower forming the air flowing passage from the air suction port to the air blow-out port in the housing, the pre-filter that is disposed on the air flowing passage, collects the easily-precipitable materials out of all the materials contained in the air introduced into the housing, which are liable to be precipitated when these materials float in the air, and pass the hardly-precipitable materials, the gas-liquid contact member that is disposed at the downstream side of the pre-filter on the air flowing passage, has the gas-liquid contact face and brings the air passed through the pre-filter into contact with the electrolytic water containing the active oxygen species on the gas-liquid contact face to remove the hardly-precipitable materials from the air, and the electrolytic water supply unit for supplying the gas-liquid contact member with the electrolytic water containing the active oxygen species.

According to this construction, the easily-precipitable materials when floating in the air are collected by the pre-filter disposed at the upstream side of the air flowing passage. Furthermore, the gas-liquid contact member disposed at the downstream side of the pre-filter, and the electrolytic water containing active oxygen species is brought into contact with the air containing the hardly-precipitable materials in the air passing through the pre-filter. Accordingly, the relatively large size (particle diameter) and easily-precipitable materials particulate materials floating in the air such as house dust such as lint, etc., pollens such as cedar pollen, etc., dust of sand, soil, etc., large fungus of spore, etc. can be collected by the pre-filter. In addition, the small size (small particle diameter) and hardly-precipitable materials floating in the air such as so-called indoor suspended particles of dust which floats indoor for a long time, floating microorganisms such as virus, etc. floating in the air while adhering to indoor suspendedparticles of dust, gaseous materials such as odor components, etc., fractured pollens floating in the air, etc. canbeefficientlyremoved (inactivated, sterilized or the like) by using electrolytic water containing active oxygen species.

Furthermore, since the easily-precipitable air-floating materials of the relatively large size (particle diameter) out of the particulate materials floating in the air are collected by the pre-filter, the air can be efficiently sucked from the air suction port and blown out from the air blow-out port by the air blower with little pressure loss. In addition, the consumption of the electrolytic water can be reduced because the large particle-size materials out of the air-floating materials are removed from the air by the pre-filter, and thus the maintenance frequency of the gas-liquid contact member can be reduced.

In the above air filtering apparatus, it is preferable that the foreign material removing mechanism (121) is equipped with a louver (121) that can close the air blow-out port (12), and the air filtering unit (40) has a gas-liquid contact member (41) that is disposed on the air flowing passage, has a gas-liquid contact face and brings air introduced into the housing into contact with electrolytic water on the gas-liquid contact face to filter the air.

According to the above air filtering apparatus, the louver is provided at the air blow-out port for blowing out air which is brought into contact with electrolytic water on the gas-liquid contact face and thus filtered, and the air blow-out port can be closed by the louver. Accordingly, when the air blow-out port is closed by the louver at the stop time of the air flowing operation, invasion of foreign materials such as pollen, etc. into the housing and flowing of unnecessary air inside and outside the housing can be suppressed.

In the above air filtering apparatus, it is preferable that the louver (121) is equipped with a driving unit (126) for turning the louver (121) with respect to the opening face of the air blow-out port (12) and also keeping the louver (121) at any angular position with respect to the opening face.

According to the above air filtering apparatus, the louver can be kept at any angular position, so that air blown out from the air blow-out port to the outside of the housing canbe rectified by the louver and the air blowing direction can be adjusted to any direction.

In the above air filtering apparatus, it is preferable that the louver (121) is equipped with a first vane (122) and a second vane (123) having such a size that the opening face of the air blow-out port (12) can be closed by the first and second vanes (122) and (123), and the first and second vanes are disposed in parallel so as to be spaced from each other at a predetermined interval by joint pins (125) driven by the driving unit (126).

It is preferable that the above air filtering apparatus is equipped with an electrolytic bath (53) for electrolyzing water to generate electrolytic water, and the air blow-out port (12) is closed by the louver (121) when electrolytic containing ozone as the active oxygen species is generated in the electrolytic bath.

According to the above air filtering apparatus, when electrolytic water containing ozone is generated in the electrolytic bath, air flow through the air blow-out port inside and outside the housing is suppressed by closing the air blow-out port with the louver, so that odor inherent to ozone can be prevented from leaking to the outside of the housing. Accordingly, when electrolytic water containing ozone is used to clean the inside of the housing, leakage of odor to a room where the air filtering apparatus is set up can be suppressed, and indoor environment can be kept comfortable.

In the above air filtering apparatus, it is preferable that the air blowing operation of the air blower (30) is stopped when electrolytic water containing ozone as the active oxygen species is generated in the electrolytic bath (53).

According to the above air filtering apparatus, when electrolytic water containing ozone is generated in the electrolytic bath, the air flow through the air blow-out port inside and outside the housing can be more surely suppressed. Therefore, when electrolytic water containing ozone is used, leakage of odor into the room where the apparatus is set up can be surely suppressed, and the indoor environment can be kept comfortable.

In the above air filtering apparatus, it is preferable that the electrolytic bath (53) has plural electrodes (for example, at least a pair of electrodes 53a, 53b)), electrolytic water containing active oxygen species is generated by applying a voltage between the electrodes (53a, 53b), and the type of the active oxygen species to be contained in the electrolytic water is switched between ozone and another type active oxygen species by changing the potential applied to the electrodes (53a, 53b).

According to the above air filtering apparatus, the active oxygen species to be generated in the electrolytic bath can be easily switched between ozone and another type active oxygen species, and also the odor inherent to ozone can be prevented from leaking to the outside of the apparatus by closing the air blow-out port with the louver. Accordingly, the electrolytic water containing ozone emitting inherent odor can be easily used with keeping comfortable the indoor environment where the apparatus is set up.

Furthermore, by changing the potential applied to the electrodes to invert the polarities of the electrodes, scales adhering to the electrodes when electrolysis is conducted exfoliate or drop off. Accordingly, the cleaning frequency of the electrodes can be reduced, and the load imposed on the maintenance work can be reduced.

According to the present invention, by providing the foreign material removing mechanism on the air flowing passage, contamination of foreign materials into the housing can be prevented, and the maintenance frequency can be reduced.

Furthermore, according to the present invention, the foreign material removing mechanism is equipped with the pre-filter for removing floating particulate materials which have a predetermined particle size or more and are easily precipitable in the air, and floating particulate materials which are smaller in particle size than the predetermined particle size and hardly precipitable in the air can be removed by using electrolytic water containing active oxygen species. Accordingly, the air-floating particulate materials containing air-floating microorganisms such as virus, etc. can be efficiently removed from the air, and the maintenance frequency can be reduced.

Still furthermore, according to the present invention, the foreign removing mechanism is equipped with the louver that can close the air blow-out port. Accordingly, invasion of foreign materials into the housing through the air blow-out port and unnecessary air flow inside and outside the housing can be suppressed, and thus the maintenance frequency can be reduced.

### BRIEF DESCRIPTION OF THE DRAWINGS

Fig. 1 is a diagram showing the construction of an air filtering apparatus according to an embodiment of the present invention;
Fig. 2 is a front view showing a gas-liquid contact member applied to an air filtering unit of the embodiment;
Figs. 3A is a diagram showing the construction of an electrolytic water supply and circulation unit of the embodiment, and Fig. 3B is a diagram showing the construction of an electrolytic bath;
Fig. 4 is a perspective view showing the outlook of the air filtering apparatus of the embodiment;
Fig. 5 is a perspective view showing the internal construction of the air filtering apparatus of Fig. 4;
Fig. 6 is a partially broken cross-sectional view showing the internal construction of the air filtering apparatus of Fig. 4;
Fig. 7 is a left-side cross-sectional view showing the internal construction of the air filtering apparatus of Fig. 4;
Fig. 8 is a right-side cross-sectional view showing the internal construction of the air filtering apparatus of Fig. 4;
Fig. 9 is a top view showing the internal construction of the air filtering apparatus of Fig. 4;
Fig. 10 is a functional block diagram showing the construction of a control system of the air filtering apparatus of Fig. 4;
Fig. 11 is a flowchart showing the details of an ozone cleaning operation; and
Fig. 12 is a flowchart showing the details of another example of the ozone cleaning operation.

### DETAILED DESCRIPTION OF THE PREFERRED EMBODIMENTS

Preferred embodiments according to the present invention will be described hereunder with reference to the accompanying drawings.

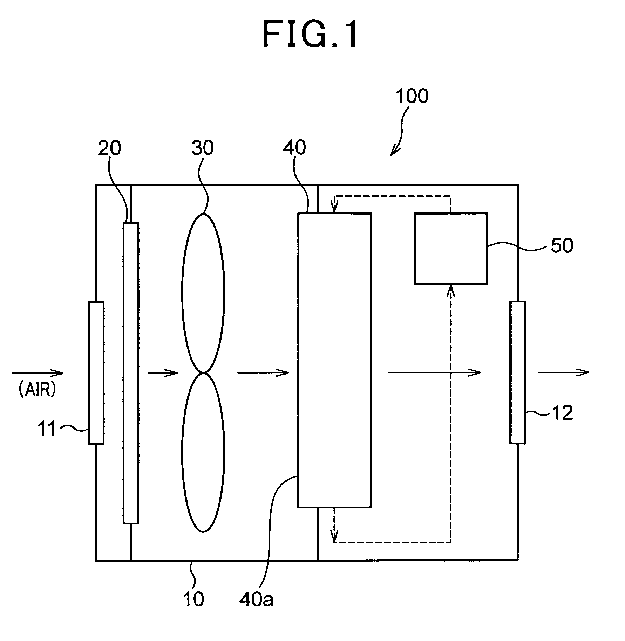

First, an air filtering apparatus 100 (air cleaning apparatus) according to the present invention will be described with reference to Fig. 1. The air filtering apparatus 100 of this embodiment has a pre-filter 20, an air blower 30, an air filtering unit 40 and an electrolytic water circulating and supply unit 50 in a housing having an air suction port 11 and an air blow-out port 12. The air blower 30 forms an air flowing passage extending from the air suction port 11 to the air blow-out port 12. The pre-filter 20 is disposed at the upstream side of the air filtering unit 40 on the air flowing passage with respect to the air flowing direction, and the air filtering unit 40 is disposed at the downstream side of the pre-filter 20 on the air flowing passage with respect to the air flowing direction.

The pre-filter 20 collects particulate materials which are relatively large in particle size (diameter) and easily precipitable when these materials float in the air (for example, particulate materials of about 10µm or more in particle size (diameter)). House dust such as lint, etc., pollens such as cedar pollen, etc., sand, dust of soil, etc. may be considered as the particulate materials as described above. The particulate materials which are easily precipitable when they float in the air will be hereinafter referred to as "easily-precipitable particulate materials".

The pre-filter 20 may be constructed by one filter or it may be constructed by combining plural filters which are different in mesh size. As an example of the combination of plural filters different in mesh size, for example, a large mesh-size filter for collecting lint, etc. is disposed at the upstream side in the air flowing direction, and a small mesh-size filter for collecting dust of soil, etc. is disposed at the downstream side in the air flowing direction. Furthermore, plural filters different in mesh size as described above are combined with one another as one assembly, and further a plurality of assemblies may be superposed on one another.

The air filtering unit 40 cleans air by removing materials which are passed through the pre-filter 20 and hardly precipitable in the air. Here, the term "removal" contains "detoxification of materials" such as inactivation, sterilization, etc. Furthermore, "particles" mean particulate small objects having observable length, width and thickness.

The air passed through the pre-filter 20 contains air-floating particulate materials which are smaller than about 10µm in particle size (hereinafter referred to as "floating microparticles"). These floating microparticles contain not only air-floating microorganisms such as virus, etc., but also so-called indoor floating dust floating in the air, gaseous materials such as odor components, etc.

The air filtering unit 40 is constructed by a gas-liquid contact member 41 having a gas-liquid contact face which is wetted with electrolytic water containing active oxygen species supplied from the electrolytic circulating and supply unit 50. Air passed through the pre-filter is brought into contact with electrolytic water on the gas-liquid contact face to remove floating microparticles such as virus, etc., thereby filtering the air. As described above, indoor air in a room where the air filtering apparatus 100 is set up is introduced into the housing 10 through the air suction port 11, particulate materials such as pollens, etc. are removed by the pre-filter 20, floating microparticles are removed in the air filtering unit 40, and then filtered and cleaned air is blown out through the air blow-out port 12 to the room.

In the air filtering apparatus 100, the location of the pre-filter 20 is not limited to a specific place insofar as it is located at the upstream side of the air filtering unit 40 on the air flowing passage formed by the air blower 30. In this embodiment, the pre-filter 20 is located at the air suction side of the air blower 30. By disposing the pre-filter at the air suction side of the air blower 30, the air blower 30 sucks air from which particulate materials such as pollens, etc. are removed, and blows the air to the air filtering unit 40.

The pre-filter 20 is designed to be larger in mesh size than HEPA filter (High Efficiency Particulate Air Filter), ULPA filter (Ultra Low Penetration Air Filter), etc., and smaller in mesh size than air filters which are normally used for pre-treatments of the HEPA filter, the ULPA filter, etc. Specifically, the mesh size of the pre-filter 20 is preferably set so that particulate materials of about 10µm or more in particle size are collected and particulate materials which are smaller than the above particle size are passed.

The pre-filter 20 may be formed of a water-washable filter. By washing the pre-filter 20 with water, collected particulate materials such as pollens, etc. can be easily removed, and clogging of the pre-filter 20 can be easily overcome.

The HEPA filter has a particle collection efficiency of 99.97% or more for particles of 0.3µm in particle diameter at a rated air flow rate, and also has an initial pressure loss of 245Pa or less (JIS Z 8122) . The pre-filter is not required to have the mesh size of the same level as the HEPA filer, and an air filter for collecting particles of about 10µm or more may be used as the pre-filter, whereby the problems of clogging and pressure loss can be overcome and a large amount of air flow can be treated.

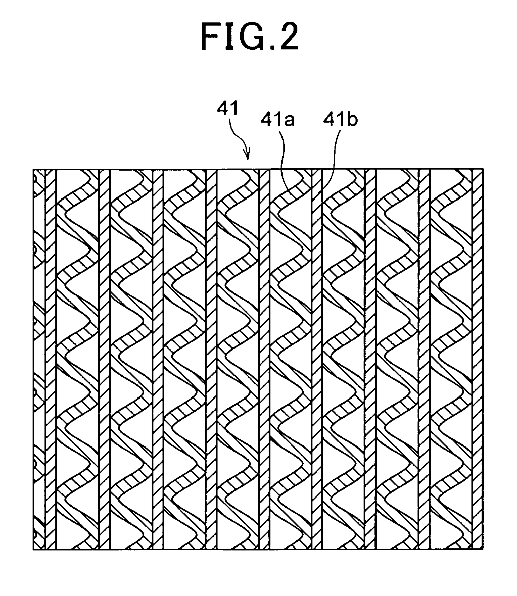

A construction shown in Fig. 2 may be applied as the gas-liquid contact member 41 constituting the air filtering unit 40. The gas-liquid contact member 41 shown in Fig. 2 is a filter member (filter element) having a honeycomb structure. In detail, the gas-liquid contact member 41 is constructed by laminating corrugated raw members 41A and flat-plate shaped raw members 41B so that a number of substantially triangular openings are formed between the corrugated raw members 41A and the flat-plate shaped raw members 41B and a honeycomb structure is formed as a whole. Accordingly, an element portion constructed by the corrugated raw members 41A and the flat-plate shaped raw members 41B is configured so that the gas-liquid contact area of the element portion when air is passed can be kept large, electrolytic water can be dropped to the element portion and clogging occurs hardly.

These raw members 41A and 41B are formed of raw material having little reactivity to electrolytic water described later, that is, rawmaterial which is little deteriorated by electrolytic water. For example, these members may be formed of polyolefin based resin (polyethylene resin, polypropylene resin or the like), PET (polyethylene terephthalate)resin, vinyl chloride resin, fluorinated resin (PTFE, PFA, ETFE or the like), cellulose based material, ceramics basedmaterial or the like. In this embodiment, PET resin is used for these raw members.

Furthermore, the gas-liquid contact member 41 may be subjected to a water affinity treatment or the like, so that the gas-liquid contact member 41 has high affinity to electrolytic water. Accordingly, the water retentivity of the gas-liquid contact member 31 to the electrolytic water (wettability) is kept, and the contact between the active oxygen species (described later) and the indoor air can be kept for a long time. Furthermore, electrolytic water having mildewproof action is dropped to the gas-liquid contact member 41, and thus breeding of fungus, etc. can be avoided without taking any mildewproof treatment (coating of mildewproof agent or the like) on the gas-liquid contact member 41, and the maintenance frequency can be reduced.

Adistributingsheet (notshown) forefficientlydispersing electrolytic water dropped from the electrolytic water circulating and supply unit 50 to the gas-liquid contact face (element portion) is disposed at the upper portion of the gas-liquid contact member 41. The distributing sheet is a sheet (woven fabric, non-woven cloth or the like) formed of textile material having liquid permeability, and one or plural distributing sheets are provided along the cross-section in the thickness direction of the gas-liquid contact member 41.

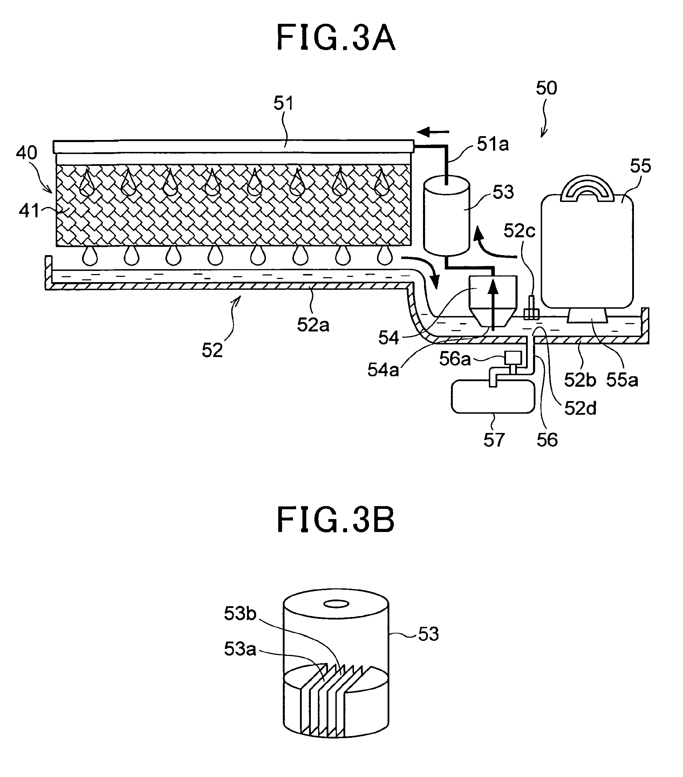

As shown in Fig. 3A, a water spray box 51 constituting a part of the electrolytic water circulating and supply unit 50 is assembled to the upper portion of the gas-liquid contact member 41, and a water receiving tray 52 constituting a part of the electrolytic water circulating and supply unit 50 is provided at the lower side of the gas-liquid contact member 41. The water receiving tray 52 receives electrolytic water discharged from the gas-liquid contact member 41.

The electrolytic water circulating and supply unit 50 is equipped with not only the water spray box 51 and the water receiving tray 52, but also an electrolytic bath 53 for generating electrolytic water containing active oxygen species, a circulating pump 54 for supplying water stocked in the water receiving tray 52 to the electrolytic bath 53, a circulating pump 54 for supplying water stocked in the water receiving tray 52, a water supply tank 55 for supplying water to the water receiving tray 52, and a drain tray 57 for draining water stocked in the water receiving tray 52 through a drain pipe 56.

The water spray box 51 is a tubular member assembled to the upper portion of the gas-liquid contact member 41, and plural water spray holes (not shown) are formed in the lower surface of the water spray box 51. The water spray box 51 is connected to the electrolytic bath 53 through an electrolytic water supply pipe 51a, and electrolytic water supplied from the electrolytic bath 53 through the electrolytic water supplypipe 51a is dropped from the water spray holes to the gas-liquid contact member 41. That is, the electrolytic water supply unit for supplying electrolytic water to the gas-liquid contact member 41 is constructed by the electrolytic bath 53, the water spray box 51, the electrolytic water supply pipe 51a, etc.

As shown in Fig. 3B, the electrolytic bath 53 has at least a pair of electrodes 53a, 53b, and when a voltage is applied between the electrodes 53a and 53b, water such as tap water or the like flowing into the electrolytic bath 53 is electrolyzed to generate electrolytic water containing active oxygen species. Here, the active oxygen species means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygen such as ozone, hypochlorous acid, hypohalous acid, etc.

The electrolytic bath 53 is preferably disposed in proximity to the gas-liquid contact member 41. By disposing the electrolytic bath 53 in proximity to the gas-liquid contact member 41, electrolytic water containing active oxygen species achieved by electrolyzing water such as tap water or the like can be immediately supplied to the gas-liquid contact member 41.

The electrode 53a may be constructed by an electrode plate comprising a base of Ti (titan) and a coated layer of Ir (iridium), Pt (platinum). By applying positive potential to this electrode as an anode from an external power supply, hypochlorous acid can be generated as an active oxygen species.

The electrode 53b may be constructed by an electrode plate comprising a base of titan and a coated layer of platinum, tantalum. By applying positive potential this electrode as an anode from the external power supply, ozone can be generated as an active oxygen species.

By applying a voltage between the electrodes 53a and 5b from the external power supply to make current flow therebetween while the electrode 53a is set to an anode and the electrode 53b is set to a cathode, hydrogen ions (H⁺) and hydroxide ions (OH⁻) in water react with each other at the electrode 53b as the cathode according to the following reaction formula (1):

4H⁺ + 4e⁻ + (4OH⁻) → 2H₂ + (4OH⁻) (1)

Furthermore, water is electrolyzed at thee electrode 53a as an anode according to the following reaction formula (2):

2H₂O → 4H⁺ + O₂ + 4e⁻ (2)

At the same time, chlorine ions (CL⁻) contained in water reacts according to the following reaction formula (3), and chlorine (Cl₂) is generated.

2 Cl⁻ → Cl₂ + 2e⁻ (3)

Furthermore, Cl₂ thus generated reacts with water according to the reaction formula (4):

Cl₂ + H₂O → HClO + HCl (4)

Hypochlorous acid generated at the electrode 53a is contained in the broadly-defined active oxygen species, and it has strong oxidizing action and bleaching action. Water solution in which hypochlorous acid is dissolved, that is, electrolytic water generated in the air filtering apparatus 100 exercises various air cleaning effects such as inactivation of virus, etc., sterilization, decomposition of organic materials, etc.

On the other hand, by applying a voltage between the electrodes 53a and 53b from the external power supply to make current flow therebetween while the electrode 53a is set to a cathode and the electrode 53b is set to an anode, the reactions indicated by the following reaction formulas (5) to (7) occurs at the electrode 53b as an anode, and ozone is generated.

2H₂O → 4H⁺ + O₂ + 4e⁻ (5)

3H₂O → O₃ + 6H⁺ + 6e⁻ (6)

2H₂O → O₃ + 4H⁺ + 4e⁻ (7)

Furthermore, at the electrode 53a as a cathode, the reactions indicated by the following reaction formulas (8) and (9) occur, and O₂⁻ generated by the electrode reaction and H⁺ in the solution are coupled to each other to generate hydrogen peroxide (H₂O₂).

4H⁺ + 4e⁻ + (4OH⁻) → 2H₂ + (4OH⁻) (8)

O₂⁻ + e⁻ + 2H⁺ → H₂O₂ (9)

As described above, in the construction of this embodiment, the voltage is applied between the electrodes 53a, 53b from the external power supply so that the electrode 53a is set to positive potential, whereby hypochlorous acid having strong sterilizing power is generated from the electrode 53a and electrolytic water containing hypochlorous acid can be generated.

Furthermore, by applying the voltage between the electrodes 53a and 53b from the external power supply so that the electrode 53b is set to positive potential, ozone having strong sterilizing power is generated from the electrode 53b side and hydrogen peroxide is generated from the electrode 53a side, so that electrolytic water containing ozone and hydrogen peroxide can be generated.

When hypochlorous acid having strong sterilizing power is generated by the electrodes 53a, 53b, electrolytic water containing hypochlorous acid is dropped from the water spraying box 51 to the gas-liquid contact member 41, and brought into contact with air blown out by the air blowing fan 31 in the gas-liquid contact member 41. At this time, virus, etc. floating in the air are inactivated, and also odor materials contained in the air react with hypochlorous acid , so that these materials are decomposed or ionized and dissolved in the electrolytic water. Accordingly, filtering (sterilization, inactivation, etc.) and deodorization of the air are performed, and cleaned air can be discharged from the gas-liquid contact member 41.

Furthermore, when ozone and hydrogen peroxide are generated by the electrodes 53a and 53b, air blown out by the air blowing fan 31 is brought into contact with ozone and hydrogen peroxide in the gas-liquid contact member 41, whereby virus, etc. floating in the air is inactivated, and also odor materials contained in the air react with ozone and hydrogen peroxide, so that these materials are decomposed or ionized and dissolved in the electrolytic water. Accordingly, filtering (sterilization, inactivation, etc.) and deodorization of the air are performed, and cleaned air can be discharged from the gas-liquid contact member 41.

The inactivating mechanism of virus, etc. by the active oxygen species will be described by exemplifying influenza virus. The active oxygen species functions to break down and vanish (remove) the surface protein (spike) of the virus concerned which is indispensable for infection. When the surface protein of influenza virus is broken down, the influenza virus is not joined to a receptor which is necessary for infection of the virus concerned, so that infection can be prevented. Therefore, influenza virus floating in the air is brought into contact with the electrolytic water containing the active oxygen species in the gas-liquid contact member 41, so that the influenza virus loses so-called infection power, and thus the infection can be prevented.

Accordingly, even when the air filtering apparatus 100 is set up in a so-called large space such as a kindergarten, an elementary/junior high/ high school, long-term care insurance facilities, a hospital or the like, air cleaned (sterilized, inactivated, deodorized, etc.) by electrolytic water is enabled to broadly go around in a large space. Therefore, air filtering and deodorization in a large space can be efficiently performed.

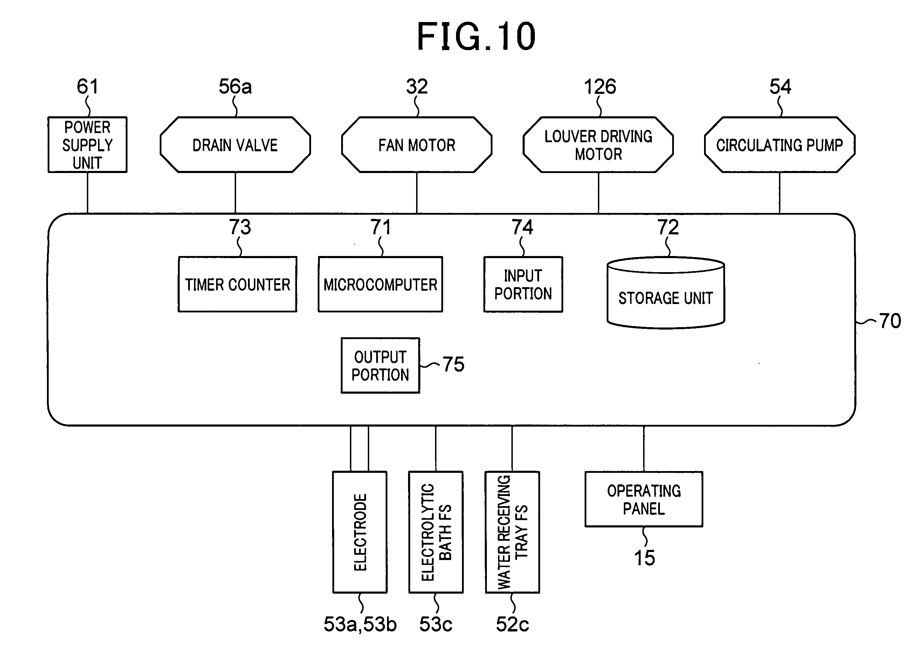

The electrode switching operation for setting any one of the electrodes 53a, 53b in the electrolytic bath 53 to positive potential can be performed by inverting the polarities of the electrodes. In this embodiment, the inversion of the electrodes can be performed by changing (inverting) the voltage applied to the electrodes 53a, 53b by a controller 70 (Fig. 10) described.

The concentration of the active oxygen species in the electrolytic water is adjusted so that virus, etc. to be filtered can be inactivated. The adjustment of the concentration of the active oxygen species is performed by adjusting the voltage applied between the electrode 53a and the electrode 53b and thus adjusting the current value flowing between the electrode 53a and the electrode 53b.

For example, when positive potential is applied to the electrode 53a and the current value flowing between the electrode 53a and the electrode 53b is set to 20mA (milliampere)/cm² in current density, a prescribed concentration of free residual chlorines (for example, 1mg/l (liter) can be generated. Furthermore, by changing the voltage applied between the electrodes 53a, 53b to increase the current value, the concentration of hypochlorous acid in electrolytic water can besettoahighvalue. With respect to ozone and hydrogen peroxide in electrolytic water, by applying positive potential to the electrode 53b and thus increasing the current value flowing between the electrodes 53a and 53b, the concentration of ozone or hydrogen peroxide in electrolytic water can be likewise increased.

Water to be introduced into the electrolytic bath 53 is not limited to tap water (city water), and it may be well water, pure water, purified water or the like. However, in order to efficiently perform electrolysis in the electrolytic bath 53, water having some degree of electrical conductivity is preferably introduced into the electrolytic bath 53. Therefore, when ion-species rare water such as well water, pure water, purified water or tap water in some districts is introduced into the electrolytic bath 53, it is preferable that prescribed ion species required to generate active oxygen species are added to such water.

For example, with respect to the construction of the electrodes 53a, 53b, when hypochlorous acid is generated as an active oxygen species from the electrode 53a side, it is assumed that chloride ions exist in water to be introduced into the electrolytic bath 53, and thus it is required to introduce water containing chloride ions into the electrolytic bath 53. Here according to Water Works Law, it is provided that tap water is disinfected with chlorine or the like to keep sanitation of tap water. Therefore, when the air filtering apparatus is designed so that tap water is introduced into the electrolytic bath 53, chloride ions have already existed in the tap water, and thus hypochlorous acid can be generated as active oxygen species with a simple construction by electrolyzing tap water in the electrolytic bath 53. At this time, when ion-species rare (diluted) water such as well water or the like is introduced into the electrolytic bath 53, chlorine compound such as salt (NaCl) or another prescribed ion species may be added into the electrolytic bath 53, whereby electrical conductivity required to perform electrolysis efficiently is achieved and also chloride ions required to generate hypochlorous acid is supplied.

In the above electrode construction, other active oxygen species can be generated by the similar reactions to the equations (3) and (4). For example, when iodide ions exist in water introduced to the electrolytic bath 53, hypoiodous acid is generated as an active oxygen species in the reactions similar to the reactions (3) and (4). That is, if halide ions exist in water introduced to the electrolytic bath 53 because of addition or contamination of halide, hypohalous acid is generated as an active oxygen species by the reactions similar to the reactions (3) and (4).

As described above, it is preferable that water containing a prescribed ion species required to generate an active oxygen species is introduced into the electrolytic bath 53. By adding ion-species rare water with a prescribed ion species required to generate an active oxygen species as described above, electrolytic water containing the active oxygen species can be generated. Accordingly, even when ion-species rare water is used in place of tap water, a sufficient air cleaning effect (inactivation of virus, etc., sterilization, deodorization, etc.) can be exercised.

Here, when the concentration of the ion species of water to be contained in electrolytic water is small, a chemical compound (halide or the like) may be supplied to water introduced into the electrolytic bath 53. For example, a chemical compound supply device for supplying the chemical compound may be provided to the air filtering apparatus 100, or the chemical compound may be directly injected into the electrolytic bath 53. Furthermore, the chemical compound may be poured into the water supply tank 55, or the chemical compound whose concentration is adjusted may be stocked in the water supply tank 55.

Here, salt or brine may be used as the chemical compound for supplying chloride ions, for example. For example, if the concentration of brine in the electrolytic bath 53 is adjusted to about 2 to 3% (weight percentage), the brine is electrolyzed in the electrolytic bath 53 to generate electrolytic water containing hypochlorous acid or hydrogen peroxide (0.5 to 1%). According to this construction, even when the ion species of water introduced into the electrolytic bath 53 is rate, the ion species can be increased by adding salt or brine, whereby the active oxygen species can be highly efficiently and stably generated through the electrolysis of water.

The water receiving tray 52 is equipped with a water receiving unit 52a located below the gas-liquid contact member 41 and also a water supply tank support unit 52b which is continuously connected to the water receiving unit 52a and located below the water supply tank 55.

The water receiving unit 52a receives water (electrolytic water) dropped from the gas-liquid contact member 41 and stocks the water therein. Accordingly, it is preferably designed to have a depth to stock a predetermined amount of water, and water received by the water receiving unit 52a is made to flow into the water supply tank support unit 52b.

The water supply tank support unit 52b is designed to have a deeper bottom than the water receiving unit 52a, and a water supply port 55a of the water supply tank 55, a suction port 54a of the circulating pump 54 and a water receiving tray float switch 52c for detecting the water level of the water supply tank support unit 52b are disposed in the water supply tank support unit 52b. The water receiving tray float switch 52c is switched to ON when the water level of the water supply tank support unit 52b is lower than a predetermined water level.

Furthermore, a float valve (not shown) is provided at the water supply port 55a of the water supply tank 55, and when the water surface of water such as electrolytic water or water such as tap water or the like stocked in the water supply tank support unit 52b is lower than the water supply port 55a, a required amount of water such as tap water or the like is supplied from the water supply tank 55, and the water level in the water supply tank support unit 52b is kept constant.

Water stocked in the water supply tank support unit 52b is pumped by the circulating pump 54, and supplied to the electrolytic bath 53. That is, water (electrolytic water) discharged from the gas-liquid contact member 41 is supplied to the electrolytic bath 53 again, whereby the use amount of tap water or the like supplied from the water supply tank 55 can be reduced. That is, according to this embodiment, water is circulated and the air filter can be efficiently performed for a long time by effectively using a small amount of water. Furthermore, when the amount of circulating water is reduced in the electrolytic water circulating and supply unit 50 due to vaporization or the like, a proper amount of water in the water supply tank 55 is supplied to the water supply tank support unit 52b.

A drain hole 52d is formed at the bottom portion of the water supply tank support unit 52b, and a drainpipe 56 is connected to the drain hole 52d. The drain pipe 56 is provided with a drain valve 56a, and water in the water supply tank support unit 52b can be discharged through the drain pipe 56 by opening the drain valve 56a. A drain tray 57 to which the drain pipe 56 is introduced is provided below the water supply tank support unit 52b, and water stocked in the water supply tank support unit 52b can be discharged through the drain pipe 56 to the drain tray 57. The drain tray 57 is formed to have a tank-like shape having a predetermined depth, and it can be taken in and out.

As described with reference to Figs. 1 to 3, according to the air filtering apparatus 100, bulky particulate materials such as pollens, etc. into which electrolytic water hardly infiltrates, out of air-floating particulate materials, are removed by the pre-filter 20, and air-floating microparticles such as air-floating microorganisms such as virus, etc. are treated in the air filtering unit 50, whereby the air filtering can be efficiently performed. Furthermore, the pre-filter 20 passes therethrough floating microparticles having particle sizes less than 10µm, whereby the clogging and pressure loss problems of the pre-filter 20 can be avoided and a large flow amount of indoor air can be treated. Furthermore, the particle materials such as pollens, etc. are collected by the pre-filter 20, whereby adhesion of stain to the gas-liquid contact member 41 can be suppressed and the air filtering can be performed stably for a long term in the gas-liquid contact member 41, and also the maintenance period of the gas-liquid contact member 41 can be lengthened. Furthermore, if the pre-filter 20 is formed of a water-washable filter, collected particulate materials such as pollens, etc. can be easily removed by washing the pre-filter with water, so that the maintenance is easy.

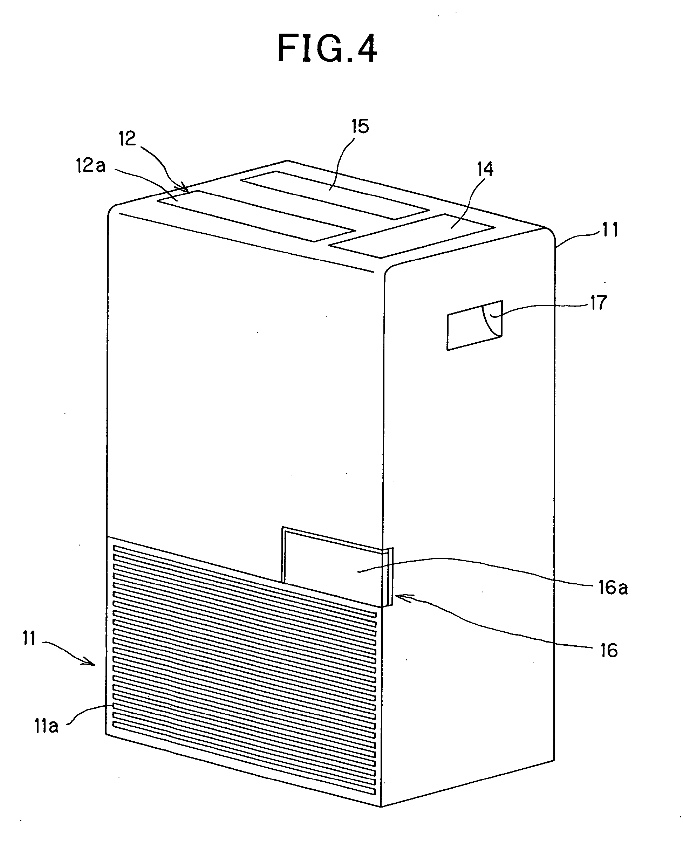

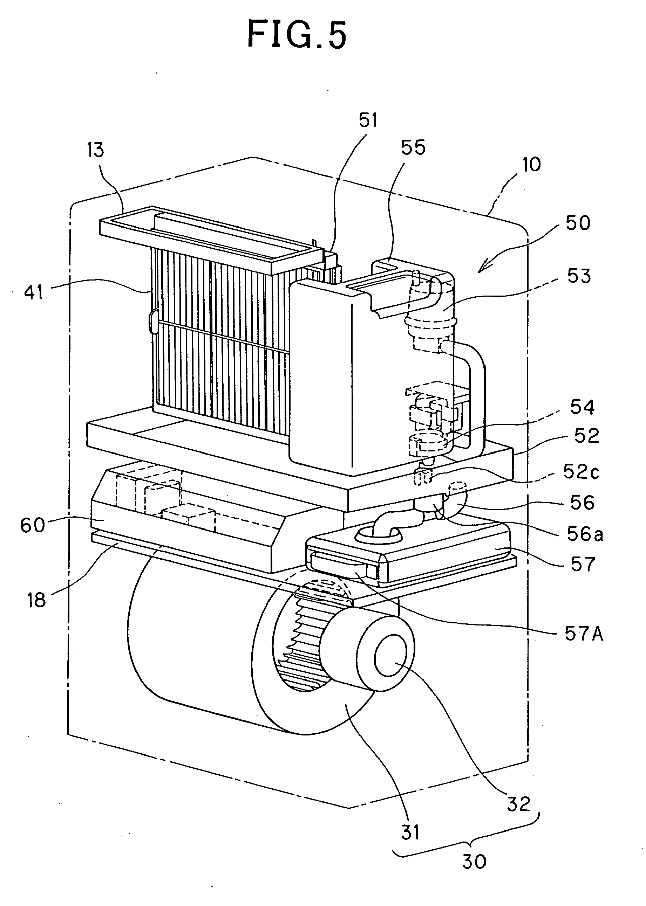

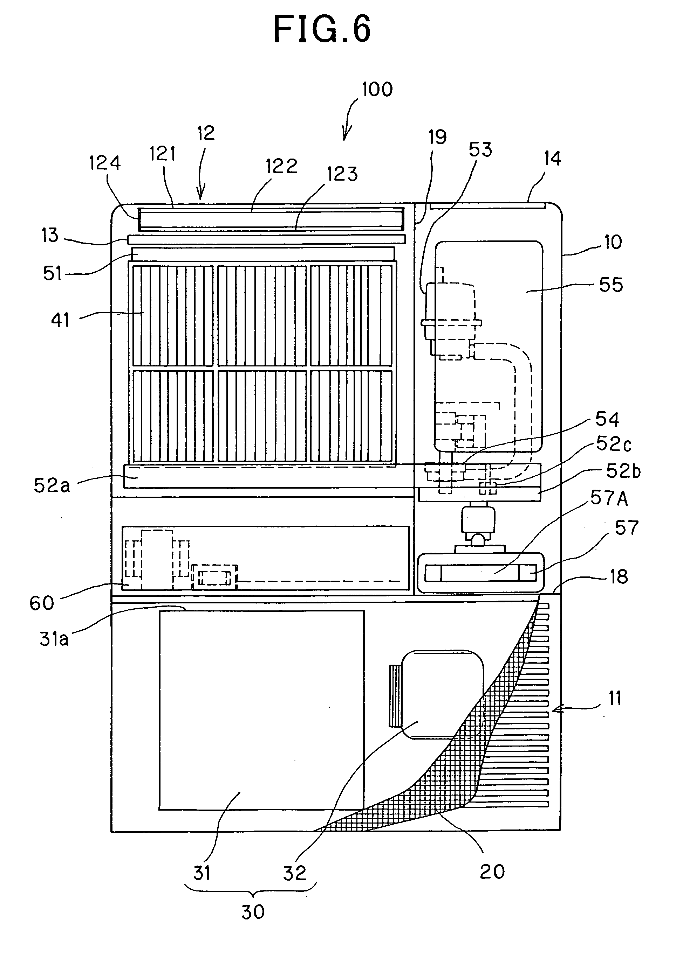

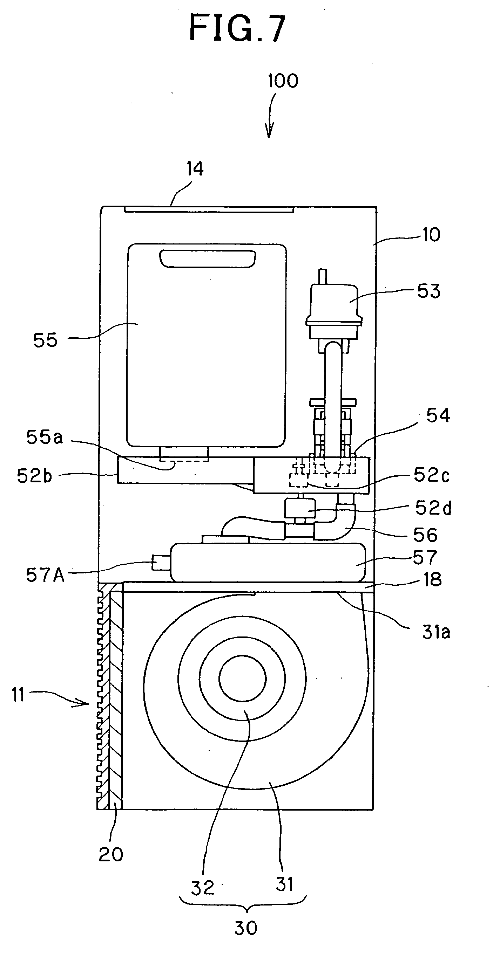

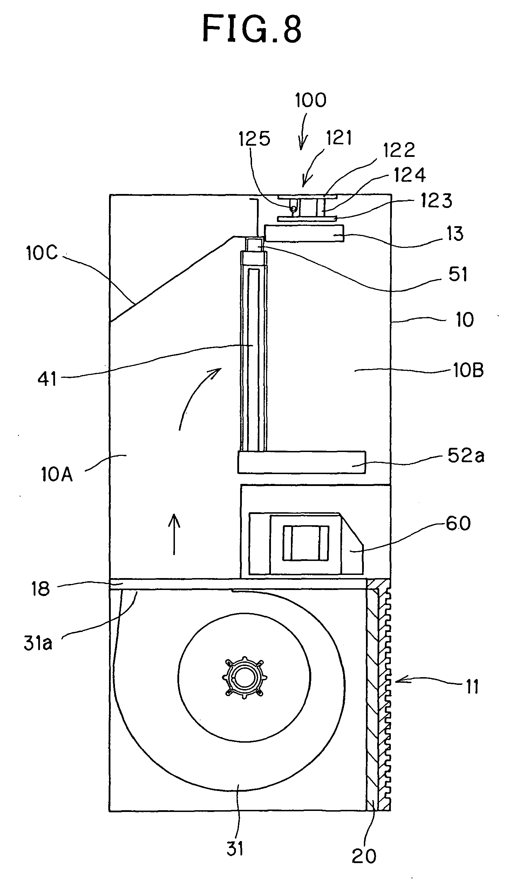

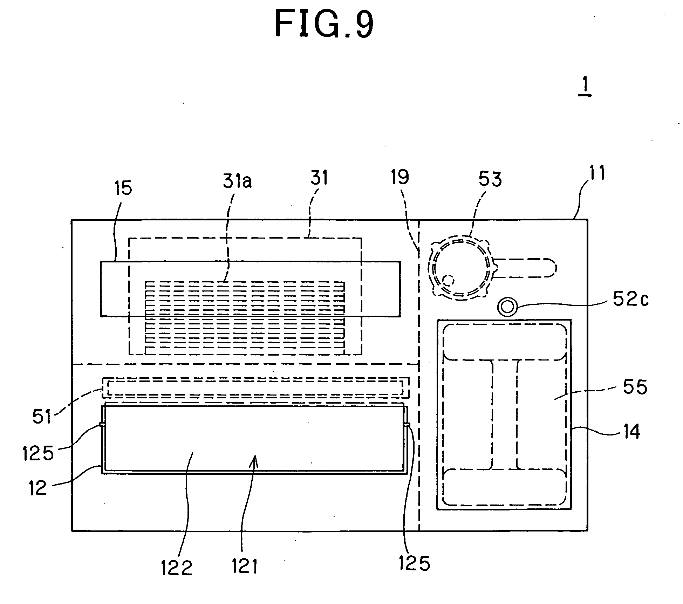

Next, a more specific construction of the air filtering apparatus 100 of this embodiment will be described. Here, Fig. 4 is a perspective view showing the outlook of the air filtering apparatus (air cleaning apparatus) 100 of this embodiment, Fig. 5 is a perspective view showing the internal construction of the air filtering apparatus 100, Fig. 6 is a partially broken front view showing the construction of the air filtering apparatus 100, Fig. 7 is a left-side cross-sectional view, Fig. 8 is a right-side cross-sectional view and Fig. 9 is a top view.

The air filtering apparatus 100 shown in Fig. 4 is an on-floor mount type, and the housing 10 is designed to have a vertically elongated box shape.

An air suction grille 11a is provided to the lower portion of the front face of the housing 10, and constitutes the air suction port 11. As shown in Figs. 6 and 7, the pre-filter 20 described above is disposed at the back surface of the air suction port 11, thereby preventing invasion of particulate materials (foreign materials) such as pollens, etc. into the housing 10.

An air blow-out port 12 is formed at the top face of the housing 10, and a louver 121 as a lid member is provided at the air blow-out port 12. This louver 121 is controlled by a controller 70 described later so that the air blow-out direction can be changed. When the air blow-out operation of the air filtering apparatus 100 is stopped, the louver 121 is closed to prevent invasion of foreign materials into the housing 10. Furthermore, an air blow-out filter 13 formed of a mesh, a woven fabric, non-woven cloth or the like (see Figs. 6 and 8) are disposed, and invasion of foreign materials such as dust, etc. into the housing 10 is prevented by the air blow-out port filter 13. Synthetic resin may be used as the material of the air blow-out port filter 13, and the same material as the gas-liquid contact member 41 is more preferably used. The air blow-out port filter 13 is preferably designed to a properly rough mesh size so that the air flowing resistance of air passed through the gas-liquid contact member 41 is not remarkably increased.
As shown in Figs. 4, 8 and 9, the louver 121 has an upper plate (first vane) 122 having such a size that it can close the air blow-out port 12, a lower plate (second vane) 123 disposed below and in parallel to the upper plate 122, a joint portion 124 for joining the upper plate 122 and the lower plate 123, etc. The joint portion 124 is a plate-shaped member provided to each of right and left end portions of each of the upper plate 122 and the lower plate 123, and pins 125 are erected from these joint portions 124. These two pins 125 are projected from both the side ends of the louver 121 to the housing 10 side, and engaged with a receiving portion (not shown) provided aside the air blow-out port to support the louver 121.

The two pins 125 are supported at the receiving portion so as to be freely swingable, and connected to a louver driving motor 12 6 (see Fig. 10) as a driving unit. The pins 12 5 are driven by the louver driving motor 126, and the louver 121 is swingable in connection with the driving of the pins 125.

Here, under the state that the louver 121 is substantially parallel to the top face of the housing 10, the air blow-out port 12 is substantially closed by the upper plate 122. This state will be referred to as "close state" of the louver 121. On the other hand, the state that the louver 121 is inclined with respect to the top face of the housing 10 will be referred to as "open state".

Under the open state of the louver 121, air passed through the gas-liquid contact member 41 can be blown out from the air blow-out port 12. Here, the louver 121 is adjusted to any angular position by the louver driving motor 126, and also held at that angular position. Therefore, the air blow-out direction from the air blow-out port 12 can be adjusted by the louver 121. The louver 121 has a two-vane structure that the upper plate 122 and the lower plate 123 are arranged in parallel to each other so as to be spaced from each other at a predetermined interval. Therefore, it has an action of rectifying air blown out from the air blow-out port 12, so that the air can be smoothly exhausted from the air blow-out port.

Furthermore, when the louver 121 is set to the close state under the state that a fan motor 32 described later is stopped, the air in the housing hardly leaks to the outside. As described later, when an active oxygen species of high concentration is generated in the electrolytic bath 53, or when ozone is generated as an active oxygen species, odor inherent to these materials occurs. However, by setting the louver 121 to the close state in such a case, the inherent odor concerned can be prevented from leaking to the outside of the housing 10. Therefore, the indoor environment under which the air filtering apparatus 100 is set up can be kept comfortable, and active oxygen species, ozone, etc. of high concentration can be used.

In addition, an opening/closing lid 14 through which water supply tank 55 disposed in the housing 10 is taken in and out, and an operating panel 15 are provided on the top face of the housing 10. As described later, the water supply tank 55 is disposed at the right side of the upper portion of the housing 10 in front view of the figure. Therefore, the opening/closing lid 14 is located at the right side on the top face of the housing 10 and also above the water supply tank. The air blow-out port 12 and the operating panel 15 are formed to be elongated in the width direction of the housing 10, and arranged in parallel to each other. The air blow-out port 12 is located at the front side on the top face of the housing 10, and the operating panel 15 is located at the back side on the top face of the housing 10.

Furthermore, a take-out port 16 of the drain tray 57 trough which the drain tray 57 is taken in and out is formed above the air suction grille 11a at the right side of the front face of the housing 10, and a take-out port cover 16a is provided at the take-out port 16. A recess portion 17 is formed at the upper portion of each of both side faces of the housing 10, and a carrier can lift up and carry the air filtering apparatus 100 by himself/herself with the recess portions 17 as grip portions.

Next, the detailed construction of each constitute element constituting the air filtering apparatus 100 and the arrangement in the housing 10 will be described with reference to Figs. 5 to 9.

As shown in Figs. 5 to 8, a partition plate 18 through which the inside of the housing 10 is divided into upper and lower chambers is provided in the housing 120. The partition plate 18 is located at the intermediate position between the air suction grille 11a and the drain tray 57, and the drain tray 57 is mounted on the partition plate 18. The drain tray 57 is provided with a knob 57A for helping user's grasp, and it can be easily taken in and out through the take-out port 16 (Fig. 4). An air blower 30 is disposed below the partition plate 18. The air blower 30 comprises an air blowing fan 31, and a fan motor 32 for driving the air blowing fan 31. Below the partition plate 18, the air blowing fan 31 is disposed at the left side of the housing 10, and the fan motor 32 is disposed at the right side of the housing 10.

The air blowing port 31a of the air blowing fan 31 is provided face up at the back side portion of the housing 10. An opening is formed in the partition plate 18 so as to be located above the air blowing port 31a of the air blowing fan 31. This opening intercommunicates with a space 10A extending vertically at the back side of the housing 10 (hereinafter referred to as "back-side space 10A"). Accordingly, indoor air sucked from the air suction port 11 by the air blowing fan 31 directs from the front face side of the lower portion of the housing 10 to the back side, and further flows upwardly in the back side space 10A of the housing 10.

A partition wall 19 for further dividing the inside of the housing to the right and left chambers is provided above the partition plate 18. The drain tray 57 is disposed above the partition plate 18 at the right side of the partition wall 19, and the water supply tank support unit 52b of the water receiving tray 52 is disposed above the drain tray 57. The water supply tank 55, the circulating pump 54 and the electrolytic bath 53 are disposed above the water supply tank support unit 52b. An opening is formed in the partition wall 19 at the position corresponding to the water receiving tray 52, and the water receiving unit 52a which is continuously connected to the water supply tank support unit 52b is disposed at the left side of the partition wall 19.

An electrical component box 60 is disposed between the partition plate 18 and the water receiving unit 52a at the left side of the partition wall 19. In the electrical component box 60 are accommodated various kinds of electrical parts such as a power supply circuit for supplying a power supply voltage to the fan motor 32, a controller 70 for controlling the respective parts of the air filtering apparatus 100, etc.

The gas-liquid contact member 41 is provided through the water receiving portion 52a of the water receiving tray 52 above the electrical component box 60 so as to be erected substantially vertically. The lower end of the gas-liquid contact member 41 is located in the water receiving unit 52a, and also the water spray box 51 is assembled to the upper portion of the gas-liquid contact member 41 as described above.

As shown in Fig. 8, the back side space 10A and a front side space 10B are formed at the left side of the partition wall 19 by the gas-liquid contact member 41.

An air guide plate 10C which slopes from the back side of the housing 10 to the front side of the housing 10 is provided above the back side space 10A, and the front end of the air guide plate 10C is connected to the water spray box 51. Air which is blown by the air blowing fan 31 is guided to the gas-liquid contact member 41 by the air guide plate 10C at the upper portion of the back side space 10A, passed through the gas-liquid contact member 41 to the front side space 10B, passed through the air blow-out port 12 formed in the top face of the housing 10 and then blown out from the air blow-out port 12.

That is, air sucked from the air suction port 11 provided at the lower portion of the front face of the housing 10 by the air blower 30 in the housing 10 temporarily flows upwardly at the lower portion of the housing 1 as indicated by an arrow of the figure, flows upwardly in the back side space 10A, passes through the gas-liquid contact member 41 to the front face side by the air guide plate 10C, and then flows to the air blow-out port 12 formed in the top face of the housing 10, thereby forming an air flowing passage.

As described above, in the on-floormount type air filtering apparatus 100, the air suction port 11 is formed at the lower portion of the housing 10, the air blow-out port 12 is formed at the upper portion of the housing 10, and the air flowing passage extending from the lower portion to the upper portion in the housing 10 is formed, whereby materials which are easily precipitable in the air such as pollens, etc., can be efficiently collected by the pre-filter 20 disposed at the back side of the air suction port 11.

Next, the operation of this embodiment will be described.

By operating the operating panel 15 shown in Figs. 4 and 9, the on-floor mount type air filtering apparatus 100 starts to operate. When the operation of the air filtering apparatus 100 is started, the circulating pump 54 is driven, and water such as tap water or the like stocked in the water supply tank support unit 52b is pumped up and supplied to the electrolytic bath 53.

In the electrolytic bath 53, a predetermined voltage is applied between the electrodes 53a and 53b, and CD current flows between the electrodes 53a and 53b, thereby electrolyzing water (tap water or the like). In this electrolysis, electrolytic water containing a predetermined active oxygen species such as hypochlorous acid or the like is generated. The electrolytic water is introduced into the electrolytic water supply pipe 51a, and dropped to the gas-liquid contact member 41 through water spray holes (not shown) of the electrolytic water supply pipe 51a. The electrolytic water is soaked to the upper edge portion of the gas-liquid contact member 41 and gradually infiltrates to the lower portion.

The air blowing fan 31 is driven by the fan motor 32, and indoor air sucked from the air suction port 11 by the air blowing fan 31 is passed from the air blowing port 31a of the air blower 30 through the air flowing passage indicated by the arrow of Fig. 8 and also through the gas-liquid contact member 41. The indoor air is brought into contact with the active oxygen species contained in the electrolytic water infiltrating into the gas-liquid contact member 41, and then blown out from the air blow-out port to the room again. At this time, with respect to the floating microparticles in the air, the virus, etc. are inactivated, and the gaseous materials such as odor materials, etc. are also dissolved in the electrolytic water or react with hypochlorous acid in the electrolytic water while passing through the gas-liquid contact member 41, whereby these materials are removed and deodorized.

On the other hand, extra electrolytic water is discharged from the gas-liquid contact member 41 and then received at the water receiving unit 52. The water stocked in the water receiving unit 52a flows into the water supply tank support unit 52b. The water stocked in the water supply tank support unit 52b is pumped and supplied to the electrolytic bath 53 by the circulating pump 54 again. When the amount of water is reduced in the water supply tank support unit 52b due to vaporization or the like, a proper amount of water such as tap water or the like is supplied from the water supply tank 55 into the water supply tank support unit 52b. When the amount of water in the water supply tank 55 is lower than a predetermined water level, the water receiving flat switch 53c is switched to ON, and a water supply lamp on the operating panel 15 or the like is turned on. In this case, a user or the like opens the opening/closing lid 14 to take out the water supply tank 55, and tap water or the like is supplemented into the water supply tank 55.

When the operation of the air filtering apparatus is finished or the maintenance work is finished, the drain valve 56a is opened, and the electrolytic water stocked in the water supply tank support unit 52b is drained to the drain tray 57. The drain tray 57 can be drawn out from the take-out port 16 and taken out to the outside of the housing 10. Accordingly, the user or the like can discard wasted water in the drain tray 57 and clean the drain tray 57.

Next, the construction of the control system of the air filtering apparatus 100 concerned will be described.

Fig. 10 is a functional block diagram showing the construction of the control system for the air filtering apparatus 100.

As shown in Fig. 10, the fan motor 32, the circulating pump 54, the drain valve 56a, the louver driving motor 126 for opening/closing the louver 121 and a power supply unit 61 for supplying power to the respective parts described above are connected to the controller 70, and operated under the control of the controller 70.

Various kinds of switches, indicator lamps (containing the water supply lamp) arranged on the operating panel 15, etc. are connected to the controller 70, and also the water receiving tray float switch 52c, the electrodes 53a, 53b and an electrolytic bath float switch 53c for detecting the water level in the electrolytic bath 53 are connected to the controller 70.

The controller 70 comprises a microcomputer 71 for controlling the overall air filtering apparatus 100, a storage unit 72 for storing a control program executed by the microcomputer 71 and data such as control parameters (for example, a set time T0 described later), etc., a timer counter 73 for carrying out time counting operation on the basis of the control of the microcomputer 71, an input portion 74 for detecting an operation on the operating panel 15 and outputting the operation content to the microcomputer 71, and an output portion 75 for outputting a processing result of the microcomputer 71 by controlling the turn-on of the indicator lamps (not shown) of the operation panel 15 or the like.

The microcomputer 71 reads out and executes the control program which is stored in the storage unit 72 in advance, and also reads out the control parameters stored in the storage unit 72. Then, the microcomputer 71 operates the respective parts of the air filtering apparatus 100 on the basis of the control program and the control parameters.

Specifically, when an operation of instructing start of the operation is carried out on the operating panel 15 and the information indicating this operation is input from the input portion 74, the microcomputer 71 operates the circulating pump 54 to start the circulation of water, and also controls the power supply unit 61 to apply a predetermined voltage between the electrodes 53a, 53b to make current flow between the electrodes 53a and 53b and generate electrolytic water.

Thereafter, the microcomputer 71 starts the operation of the fan motor 32 to start the air blowing operation of the air blowing fan 31. Through the above series of operations, the air filtering operation of the air filtering apparatus 100 is started. In connection with the start of the air filtering operation, the microcomputer 71 controls the output portion 75 to display that the air filtering apparatus is under operation.

During the time period when the air filtering operation is executed, the microcomputer 71 drives the louver driving motor 126 according to the operation on the operation panel 15 to adjust the angle of the louver 121. Furthermore, the microcomputer 71 identifies the concentration of electrolytic water in the electrolytic bath 53 (the concentration of the active oxygen species) on the basis of the electrical conductivity between the electrodes 53a and 53b, and properly adjusts the voltage applied between the electrodes 53a and 53b.

Furthermore, when the water level in the electrolytic bath 53 is detected to be lower than a predetermined water level during the execution of the air filtering operation of the air filtering apparatus 100 by the electrolytic bath float switch 53c or when the water level of the water receiving tray 42 is detected to be lower than a predetermined water level by the water receiving tray float switch 52c, the microcomputer 71 stops the application of the voltage to the electrodes 53a and 53b, and also the operation of the circulating pump 54 and the fan motor 32 is stopped. In addition, the microcomputer 71 controls the output portion 75 to display a warning.

Furthermore, when the operation of instructing the stop of the operation is carried out on the operating panel 15 and the information indicating this operation is input from the input portion 74, the microcomputer 71 stops the application of the voltage to the electrodes 53a and 53b and also stops the circulating pump 54. Furthermore, the microcomputer 71 stops the fan motor 32 to stop the air blowing operation of the air blowing fan 31, and also stops the display of the output portion 75 indicating that the air filtering apparatus is under operation. Through the above series of operations, the air filtering operation of the air filtering apparatus 100 is stopped.

When the air filtering operation of the air filtering apparatus 100 is stopped, the microcomputer 71 controls the timer counter 73 to start counting of an operation stop time (stop time T). The timer counter 73 can accumulatively count the stop time T. When the air filtering apparatus 100 starts the air filtering operation, the timer counter 73 stops the time counting operation. However, when the air filtering apparatus 100 stops the air filtering operation again, the timer counter 73 resume the counting operation from the count value before the operation is started. When the microcomputer 71 instructs to reset the timer counter 73, the timer counter 73 resets the count value.

Furthermore, the microcomputer 71 executes an ozone cleaning operation in which electrolytic water containing ozone is generated in the electrolytic bath 53 and circulated in the air filtering apparatus 100.

In the air filtering operation, the microcomputer 71 applies a voltage between the electrodes 53a and 53b so that the electrode 53a of the electrolytic bath 53 is set to the positive potential, and hypochlorous acid having strong sterilizing power is generated from the electrode 53a side. On the other hand, during ozone cleaning operation, the microcomputer 71 inverts the polarities of the electrodes and applies a voltage to the electrodes so that the electrode 53b is set to the positive potential. Accordingly, ozone having strong sterilizing power is generated from the electrode 53b side, and hydrogen peroxide is generated from the electrode 53a side, whereby electrolytic water containing ozone is circulated in the electrolytic water circulating and supply unit 50. In order to prevent the gas-liquid contact member 41 from being dried and also suppress breeding of fungus, virus, etc. in the gas-liquid contact member 41, the ozone cleaning operation is executed every predetermined time while the operation of the air filtering apparatus 100 is stopped.

The ozone cleaning operation will be described.

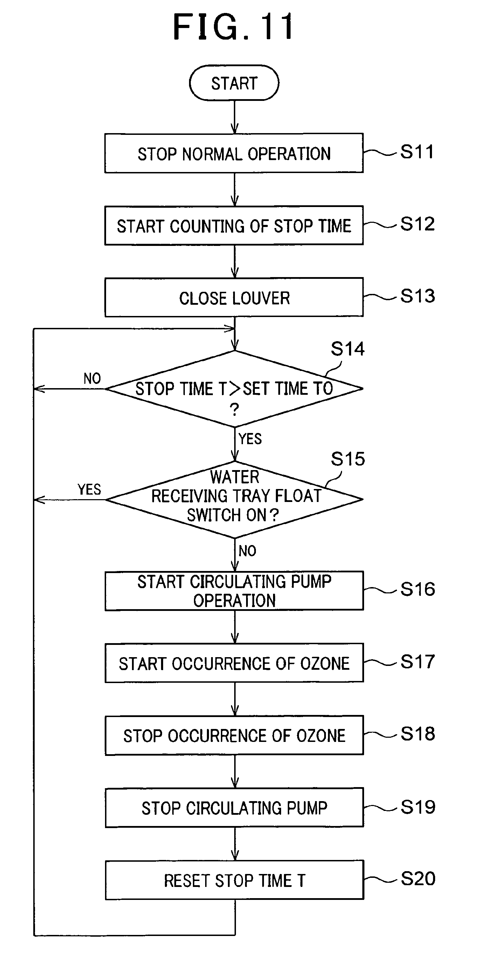

Fig. 11 is a flowchart showing the ozone cleaning operation.

First, when the operation of the air filtering apparatus 100 is stopped (step S11), the microcomputer 71 controls the timer counter 73 to start the counting of the stop time T of the air filtering apparatus 100 (step S12).

Subsequently, the microcomputer 71 drives the louver driving motor 126 to set the louver 121 to the close state (step S13), and monitors the stop time T counted by the timer counter 73 (step S14). Here, when the stop time T exceeds a set time T0 stored in the storage unit 72 in advance (step S14: Yes), the microcomputer 71 detects the state of the water receiving tray float switch 52c (step S15).

When the water receiving tray float switch 52c is set to ON (step S15: Yes), that is, the water level of the water receiving tray 42 is low, the present situation is improper to the ozone cleaning operation, and thus the processing returns to step S14.

When the water receiving tray float switch 52c is not set to ON (step S15: No), the microcomputer 71 starts the operation of the circulating pump 54 (step S16), and then applies a voltage to the electrodes 53a and 53b of the electrolytic bath 53 to generate ozone (step S17).

Accordingly, electrolytic water containing ozone in the electrolytic bath 53 is generated, and this electrolytic water is circulated in the electrolytic water circulating and supply unit 50, so that the gas-liquid contact member 41 is prevented from being dried, virus, etc. adhering to the respective parts of the electrolytic waster circulating and supply unit 50 containing the gas-liquid contact member 41 are inactivated, various bacteria, etc. are sterilized, foreign materials such as odor components, etc. are decomposed, etc., thereby keeping cleanness of the electrolytic water circulating and supply unit 50.

When the microcomputer 71 continues the operation for a predetermined time or a time indicated by the operation of the operating panel 15 from the start time of the application of the voltage to the electrodes 53a, 53b, stops the voltage application (step S18) and stops the circulating pump 54 (step S19). Thatis,electrolytic water containing ozoneiscirculated in the electrolytic water circulating and supply unit 50 for a predetermined time or for only the time indicated by the operation of the operating panel 15.

Thereafter, the microcomputer 71 resets the stop time T counted by the timer counter 73 (step S20). The timer counter 73 starts to count the stop time from an initial value (for example, zero), and the microcomputer 71 returns to the operation of step S14.

As described above, according to the air filtering apparatus 100 of this embodiment, the louver 121 is provided to the air blow-out port 12 for discharging air blown by the air blowing fan 31, and the louver 121 is designed so as to open/close the air blow-out port 12. Therefore, if the air blow-out port 12 is closed at the stop time of the air blowing fan 31, invasion of foreign materials and flow of unnecessary air through the air blow-out port 12 can be surely suppressed.

By generating electrolytic water containing ozone and circulating the electrolytic water in the electrolytic water circulating and supply unit 50 at the stop time of the air filtering operation, the respective parts of the electrolytic water circulating and supply unit 50 are sterilized/deodorized by the strong oxidizing action of ozone, thereby keeping the clean state.

Furthermore, electrolytic water containing hypochlorous acid is generated in the electrolytic bath 53 under the normal air filtering operation. Under the ozone cleaning operation, the polarities of the electrodes are inverted, and electrolytic water containing ozone and hydrogen peroxide is generated. Therefore, the respective parts of the electrolytic water circulating and supply unit 50 are cleaned by the different kind of active oxygen species from that used under the air filtering operation, so that the cleanness of the electrolytic water circulating and supply unit 50 can be more surely kept.

Furthermore, ozone is gas having strong inherent odor. Accordingly, even when the amount of ozone is minute to the extent that it does not affect the human body, the odor of ozone may make users feel uncomfortable. However, according to this embodiment, the louver 121 is set to the close state in advance before the ozone cleaning operation is carried out. Therefore, the odor of ozone hardly leaks to the outside of the air filtering apparatus 100, and thus the indoor environment in which the air filtering apparatus 100 is set up can be kept comfortable. Furthermore, the fanmotor 32 is also stoppedduring ozone cleaning operation, and the air blowing operation of the air blowing fan 31 is stopped. Therefore, the leakage of the odor of ozone to the outside of the housing 10 can be more surely and effectively suppressed.

Still furthermore, according to this embodiment, the kind of the active oxygen species contained in the electrolytic water can be easily switched to another one by inverting the polarities under ozone cleaning operation. Therefore, electrolytic water containing ozone and electrolytic water containing another kind of active oxygen species can be readily used without complicating the construction of the air filtering apparatus 100 needlessly.

In addition, by inverting the polarities of the electrodes 53a and 53b during ozone cleaning operation, scales deposited on the cathode exfoliate/fall off from the electrodes during the air filtering operation.

During the air filtering operation, scales (for example, calcium-based scales such as calcium carbonate, etc., magnesium-based scales such asmagnesiumcarbonate, etc.) derived from inorganic materials (containing ions) contained in water introduced to the electrolytic bath 53 are particularly deposited on the surface of the electrode at the cathode. When the scales are deposited on the electrode, the electrical conductivity is lowered, and thus it is difficult to continue the electrolysis. However, according to this embodiment, by inverting the polarities of the electrodes during ozone cleaning operation, the scales deposited on the electrode fall off, and thus the electrolysis efficiency in the electrodes 53a, 53b can be prevented from being lowered or the like. Accordingly, the maintenance frequency containing the cleaning of the electrodes 53a, 53b can be greatly reduced, and the labor and cost imposed on the maintenance can be greatly reduced.

In the above embodiment, the ozone cleaning operation is executed every predetermined set time under the state that the normal air filtering operation is stopped. However, the present invention is not limited to this embodiment, and for example, the ozone cleaning operation may be started according to the operation of the operating panel 15 even under the state that the air filtering operation is executed.

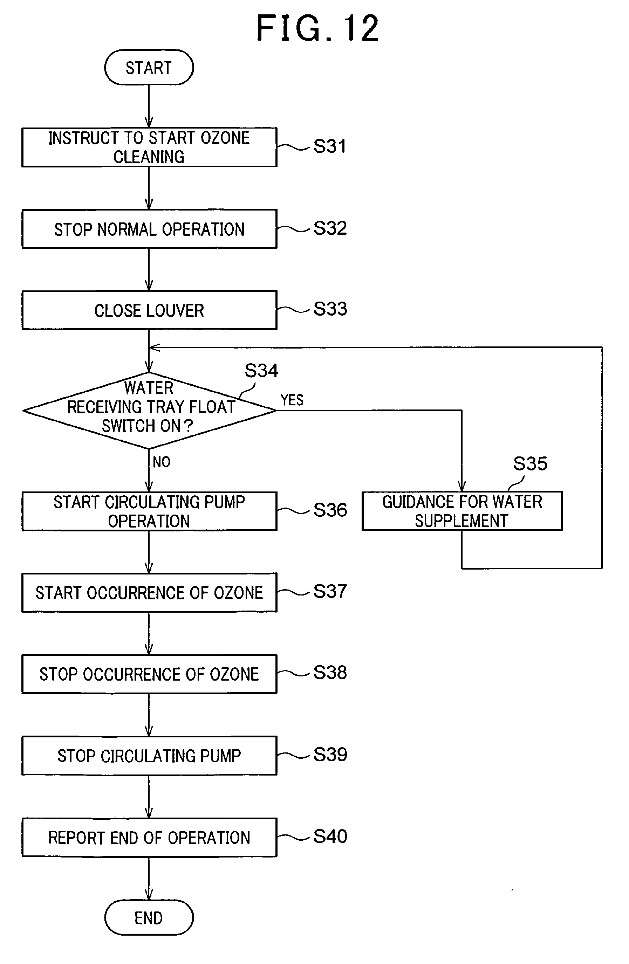

This case will be described with reference to Fig. 12.

Fig. 12 is a flowchart showing another example of the ozone cleaning operation. When the ozone cleaning operation is instructed to start by the operation of the operating panel 15 during the air filtering operation of the air filtering apparatus 100 (step S31), the microcomputer 71 stops the fan motor 32 and the circulating pump 54, and the application of the voltage to the electrodes 53a and 53b is stopped, thereby stopping the air filtering operation (step S32).

Subsequently, the microcomputer 71 drives the louver driving motor 126 to set the louver 121 to the close state (step S33), and detects the state of the water receiving tray float switch 52c (step S34).

When it is detected that the water receiving tray float switch 52c is ON (step S34: Yes), the microcomputer 71 controls the output portion 75 to output guidance indicating that water should be supplemented from the water supply tank 55 (step S35), and then returns to step S34.

When it is detected that the water receiving tray is not ON (step S34: No), the microcomputer 71 starts the operation of the circulating pump 54 (step S36), and subsequently applies the voltage to the electrodes 53a and 53b of the electrolytic bath 53 to generate ozone (step S37).

Accordingly, electrolytic water containing ozone is generated in the electrolytic bath 53, and this electrolytic water is circulated in the electrolytic water circulating and supply unit 50. Therefore, the gas-liquid contact member 41 can be prevented from being dried, the virus, etc. adhering to the respective parts of the electrolytic water circulating and supply unit 50 containing the gas-liquid contact member 41 are inactivated, various bacteria, etc. are sterilized, foreign materials such as odor components, etc. are decomposed, etc., so that the cleanness of the electrolytic water circulating and supply unit 50 can be kept.

The microcomputer 71 continues the operation for only a predetermined time or only a time indicated by the operation of the operating panel 15 from the start time of the application of the voltage to the electrodes 53a and 53b. Thereafter, the microcomputer 71 stops the application of the voltage (step S38) and stops the circulating pump 54 (step S39). That is, electrolytic water containing ozone is circulated in the electrolytic water circulating and supply unit 50 for only the predetermined time or only the time indicated by the operation of the operating panel 15.

Thereafter, the microcomputer 71 controls the output portion 75 to report that the ozone cleaning operation is finished (step S40), stops all the operations and shifts to the stop state.

In the example of Fig. 12, the ozone cleaning operation is executed at any timing by the operation of the operating panel 15, and thus the maintenance of the air filtering apparatus 100 such as cleaning of the electrolytic water circulating and supply unit 50 by the electrolytic water containing ozone, removable of scales of the electrodes 53a, 53b, etc. can be performed.

In the air filtering apparatus 100 according to this embodiment, the microcomputer 71 may control the louver driving motor 126 to set the louver 121 to the open state at the start time of the air filtering operation and set the louver 121 to the close state at the stop time of the air filtering operation.

In place of the above ozone cleaning operation, electrolytic water containing the same active oxygen species as that under the normal air filtering operation may be generated and circulated in the electrolytic water circulating and supply unit 50 for the cleaning operation. In this case, in order to exercise a higher sterilizing effect, etc. as compared with the air filtering operation, the current to be supplied to the electrodes 53a and 53b is preferably controlled to generate the active oxygen species of higher concentration. Furthermore, by setting the louver 121 to the close state and stopping the fan motor 32, odor occurring in connection with generation of the active oxygen species (hypochlorous acid or the like) of high concentration can be prevented from leaking to the outside of the housing 10, so that the cleanness of the air filtering apparatus can be kept while the indoor environment under which the air filtering apparatus 100 is set up is kept comfortable.

The present invention is not limited to the above embodiments, and various modifications may be properly made to the above air filtering apparatus 100 without departing from the subject matter of the present invention.

For example, in the illustrations shown in the figures, the electrodes 53a and 53b are paired, and two pairs of the electrodes 53a and 53b are provided. Furthermore, the polarities of these electrodes 53a and 53b are inverted to switch the type of the active oxygen species contained in the generated electrolytic water between hypochlorous acid and ozone, hydrogen peroxide. However, the construction of the electrodes provided to the electrolytic bath 53 is not limited to the above construction.

That is, the above construction may be modified so that two electrodes 53a are connected to each other in parallel and one electrode 53b is used so that three electrodes are disposed in the electrolytic bath 53. Furthermore, the above construction may be modified so that two third electrodes to which negative potential is applied at all times are provided in the electrolytic bath 53, and the electrode 53a, 53b and the two third electrodes are paired. In this case, a voltage is applied between the electrode 53a and the third electrode and between the electrode 53b and the third electrode to electrolyze water. Here, the third electrodes may be formed of platinum, carbon (C), stainless (Fe-Cr-(Ni) alloy) or the like.

Furthermore, a plurality of electrolytic baths 53 may be provided. In this case, the electrode 53a and a third electrode are inserted as an pair electrode in one electrolytic bath 53, and the electrode 53b and a third electrode are inserted as a pair electrode in another electrolytic bath 53. At this time, the electrolytic bath 53 in which water such as tap water or the like is electrolyzed is properly switched between these electrolytic baths 53, whereby the type of the active oxygen species to be contained in electrolytic water dropped to or infiltrated to the gas-liquid contact member 41 is switched.

As described above, the third electrode may be newly provided so that the electrode to be paired with the third electrode is switched between the electrodes 53a and 53b. In this case, it is preferable that the electrode to be paired with the third electrode is alternately switched between the electrode 53a and the electrode 53b. It is particularly preferable that hypochlorous acid and ozone are generated as the active oxygen species by switching the electrode to be paired with the third electrode between the electrode 53a and the electrode 53b. The shape of the electrodes is not limited to a specific one, and it may be a plate-like shape, a rod-like shape or the like.

In the above embodiment, the type of the active oxygen species generated by the electrolysis of water or the like is switched by changing the potential applied to the electrodes 53a, 53b. For example, hypohalous acid such as hypochlorous acid or the like may be mainly generated.

In this case, each of the electrodes 53a and 53b may be constructed by an electrode plate comprising a base of Ti (titan) and a coated layer of Ir(iridium), Pt (platinum).

As described above, by supplying current to tap water or the like through the electrodes 53a and 53b, the following reaction occurs at the cathode:

4H⁺ + 4e⁻ + (4OH⁻) → 2H₂ + (4OH⁻)

Furthermore, the following reaction occurs at the anode:

2H₂O → 4H⁺ + O₂ + 4e⁻

At the same time, chlorine ions (Cl⁻) contained in water (which are contained in tap water or the like in advance) reacts as follows:

2Cl⁻ → Cl₂ + 2e⁻

Furthermore, Cl₂ thus generated reacts with water:

Cl₂ + H₂O → HClO + HCl

In this construction, by supplying current to the electrodes 53a and 53b, HClO (hypochlorous acid), etc. are generated. The electrolytic water containing this hypochlorous acid, etc. is supplied to the gas-liquid contact member 41 to prevent breeding of bacteria, etc. in the gas-liquid contact member 41, the virus, etc. floating in the air passing through the gas-liquid contact member 41 can be inactivated and sterilized. Furthermore, gaseous materials such as odor components, etc. are dissolved in the electrolytic water, and react with hypochlorous acid or the like in the electrolytic water when they are passed through the gas-liquid contact member 41, so that these materials are removed from the air or deodorized.

In the above embodiment, the active oxygen species are generated by using chloride ions contained in electrolytic water by electrolyzing tap water in the electrolytic bath 53. However, the above embodiment may be modified so that an ozone generating device for generating ozone by air discharging is provided and ozone generated by the ozone generating device is dissolved in water and supplied to the gas-liquid contact member 41. In this case, the ozone generating device may directly supply the generated ozone to the electrolytic bath 53, or ozone generated in the ozone generating device may be supplied to electrolytic water generated in the electrolytic bath 53 and dissolved in the electrolytic water. According to such a construction, even when the ion species of water to be introduced to the electrolytic bath 53 is rare and thus it is difficult to generate ozone by electrolyzing the water, electrolytic water containing ozone as the active oxygen species can be generated and dropped to/infiltrated in the gas-liquid contact member 41.

Furthermore, in the above embodiment, the water supply system based on the water supply tank 55 which can be freely taken in and out is adopted. However, in place of the water supply tank 55, for example, a water distribution pipe system in which a water pipe is connectedto the air filtering apparatus to directly lead city water may be adopted.

Still furthermore, in the above embodiment, the electrolytic water is dropped to the gas-liquid contact member 41. However, the present invention is not limited to this embodiment, and the electrolytic water may be sucked up by the gas-liquid contact member 41. In this case, the lower edge portion of the gas-liquid contact member 41 is located to be lower than the water level of the electrolytic water in the water receiving tray 42 in which the electrolytic water is stocked, and the lower portion of the gas-liquid contact member 41 is submerged in the electrolytic water. Accordingly, the electrolytic water is sucked up by a so-called capillary phenomenon, whereby the electrolytic water is soaked to the gas-liquid contact member 41.

Still furthermore, it is preferable that the pre-filter is formed of a water-washable filter because the materials collected by the pre-filter can be more easily removed from the pre-filter by washing the pre-filter with water.

## Claims

1. An air filtering apparatus (100) for filtering air by using electrolytic water comprises:
a housing (10) having an air suction port (11) and air blow-out port (12);
an air blower (30) forming an air flowing passage extending from the air suction port (11) to the air blow-out port (12) in the housing;
an air filtering unit (40) that is disposed on the air flowing passage and brings air introduced into the housing (10) into contact with electrolytic water to filter the air; and
a foreign material removing mechanism (20, 121) that is disposed on the air flowing passage and removes foreign materials contained in the air.

2. The air filtering apparatus according to claim 1, wherein the foreign material removing mechanism (20) is equipped with a pre-filter (2) for collecting materials each of which is contained in air introduced into the housing and has a predetermined particle size or more, and the air filtering unit (40) has a gas-liquid contact member (41) that is disposed at the downstream side of the pre-filter (20) on the air flowing passage and has a gas-liquid contact face, the air from which the materials each having the predetermined particle size or more are removed beingbrought into contact with the electrolytic water to thereby filter the air.

3. The air filtering apparatus according to claim 3, wherein the predetermined particle size is preferably equal to about 10µm. That is, the particle passing size (diameter) of the pre-filter is preferably set to about 10µm or less.

4. The air filtering apparatus according to claim 2, wherein the housing (10) is configured as an on-floor mount type, the air suction port (11) is formed at the lower portion of the housing and the air blow-out port (12) is formed at the upper portion of the housing.

5. The air filtering apparatus according to claim 2, wherein the pre-filter (20) is disposed inside the air suction port (11).

6. The air filtering apparatus according to claim 2, wherein the pre-filter (20) comprises a water-washable filter so that collected foreign materials are removed.

7. The air filtering apparatus further comprising an electrolytic water supply unit (50) for supplying electrolytic water to the gas-liquid contact face of the gas-liquid contact member (41).

8. The air filtering apparatus according to claim 7, wherein the electrolytic water supply unit (50) is equipped with an electrolytic bath having at least a pair of electrodes (53a, 53b) for electrolyzing water or water containing predetermined ion species introduced into the electrolytic bath to generate electrolytic water containing active oxygen species.

9. The air filtering apparatus according to claim 8, wherein the active oxygen species contains at least one material selected from the group consisting of hypochlorous acid, hypoiodous acid, ozone and hydrogen peroxide.

10. The air filtering apparatus according to claim 1, wherein the foreign material removing mechanism (20) has a pre-filter through which easily-precipitable materials out of all materials contained in air introduced into the housing are collected and hardly-precipitable materials out of all the materials are passed; and the air filtering unit (40) has a gas-liquid contact member (41) that is disposed at the downstream side of the pre-filter(20) on the air flowing passage, has a gas-liquid contact face and brings air passed through the pre-filter into contact with the electrolytic water on the gas-liquid contact face, thereby removing the hardly-precipitable materials from the air.

11. The air filtering apparatus according to claim 1, wherein the foreign material removing mechanism (121) is equipped with a louver (121) that can close the air blow-out port (12), and the air filtering unit (40) has a gas-liquid contact member (41) that is disposed on the air flowing passage, has a gas-liquid contact face and brings air introduced into the housing into contact with electrolytic water on the gas-liquid contact face to filter the air.

12. The air filtering apparatus according to claim 11, wherein the louver (121) is equipped with a driving unit (126) for turning the louver (121) with respect to the opening face of the air blow-out port (12) and also keeping the louver (121) at any angular position with respect to the opening face.

13. The air filtering apparatus according to claim 12, wherein the louver (121) is equipped with a first vane (122) and a second vane (123) having such a size that the opening face of the air blow-out port (12) can be closed by the first and second vanes (122) and (123), and the first and second vanes are disposed in parallel so as to be spaced from each other at a predetermined interval by joint pins (125) driven by the driving unit (126).

14. The air filtering apparatus according to claim 11, further comprising an electrolytic bath (53) for electrolyzing water to generate electrolytic water, and the air blow-out port (12) is closed by the louver (121) when electrolytic containing ozone as the active oxygen species is generated in the electrolytic bath.

15. The air filtering apparatus according to claim 14, wherein the air blowing operation of the air blower (30) is stopped when electrolytic water containing ozone as the active oxygen species is generated in the electrolytic bath (53).

16. The air filtering apparatus according to 14, wherein the electrolytic bath (53) has plural electrodes (53a, 53b)), electrolytic water containing active oxygen species is generated by applying a voltage between the electrodes (53a, 53b), and the type of the active oxygen species to be contained in the electrolytic water is switched between ozone and another type active oxygen species by changing the potential applied to the electrodes (53a, 53b).
